# EUROPEAN PATENT APPLICATION

(11) **EP 4 599 787 A1**
(43) Date of publication of application: **13.08.2025**
(21) Application number: 23875119.2
(22) Date of filing: 20.09.2023
(51) Int. Cl.: A61B 50/20, A61B 90/00, A61B 34/30, A61B 34/00, A61B 17/00

(54) **IMPACT-REDUCING DEVICE FOR MEDICAL TOOLS AND MEDICAL HOLDER HAVING SAME**

(30) Priority: 05.10.2022 KR 20220126763
(71) Applicant: Curexo, Inc., Seoul 05814 (KR)
(72) Inventor: CHO, Gyu Il, Seoul 05814 (KR); LIM, Heung Soon, Seoul 05814 (KR)
(74) Representative: Stöckeler, Ferdinand
(86) International application number: PCT/KR2023/014305
(87) International publication number: WO 2024/076052

(57) **Abstract**

The disclosure relates to an impact-reducing device for medical tools and a medical holder having the same, and more specifically to an impact-reducing device for medical tools, which absorbs and buffers an abnormal impact applied to the medical tools to prevent a medical holder, the medical tool or peripheral devices from being deformed or damaged, and the medical holder having the same.

The impact-reducing device for medical tools, includes: an impact-reducing device main body formed with a tool insertion hole in which a tool shaft of the medical tool is inserted; an impact absorbing member placed in the impact-reducing device main body and absorbing impact applied to the medical tool; and a separation preventing member installed in the impact-reducing device main body to prevent separation of the impact absorbing member.

## Description

### [TECHNICAL FIELD]

The disclosure relates to an impact-reducing device for medical tools and a medical holder having the same, and more specifically to an impact-reducing device for medical tools, which absorbs and buffers an abnormal impact applied to the medical tools to prevent a medical holder, the medical tool or peripheral devices from being deformed or damaged, and the medical holder having the same.

### [BACKGROUND ART]

In general, surgical treatment for joint diseases includes arthroscopic surgery, chondrocyte transplantation, etc. and includes artificial joint surgery for severe conditions. As representative artificial joint surgery, there are manual artificial joint surgery manually performed by medical personnel, and robotic artificial joint surgery performed by a robot.

The robotic artificial joint surgery refers to a surgical procedure of placing an artificial joint (or implant) after cutting a bone from a joint area by rotating a cutter of a cutting device mounted to a distal end of a position-variable arm of the robot based on information input to a computer, and employs a cutting tool such as a drill, a reamer for widening a drilled hole, and an impactor for providing a press-fit force.

The artificial joint surgery is common in artificial knee joint surgery, and artificial hip joint surgery has recently been also gradually increasing.

The artificial hip joint surgery is usually performed by preprocessing an acetabulum of a hip joint using the reamer, inserting an artificial acetabulum-cup, pressing and fitting the artificial acetabulum-cup using the impactor, and then connecting an artificial femoral head.

As described above, the impactor used in the artificial hip joint surgery includes a straight impactor (or straight impactor handle), the outer appearance of which is shaped to have a straight structure, and a curved impactor (or curved impactor handle), the outer appearance of which is shaped to have a curved structure.

For example, the straight impactor includes an impactor shaft formed in a roughly rod shape, and a cap or disc-shaped striking portion formed at the rear of the impactor shaft.

The impactor is used in such a manner that the impactor shaft is inserted and held in the holder and then the striking portion is forcibly struck with a hammer to press and fit the artificial acetabulum-cup.

In the foregoing process of striking the impactor to perform a procedure by a medical man, if the striking direction of the hammer is misaligned with the longitudinal direction, i.e., axial direction of the impactor to apply an impact force to the impactor in an oblique direction (i.e., wrong direction), the holder and the impactor may be broken or damaged.

In addition, when the hammer misses the impactor, the impact force is obliquely applied even to the artificial acetabulum-cup, thereby not only having an adverse effect on the surgery but also causing injury or damage to a medical man (or robot) as the impact force of the hammer is transmitted to the medical man who (or the robot that) holds the holder.

### [Documents of Related Art]

### [Patent Documents]

(Patent document 1) Korean Patent Publication No. 10-2020-0115518, titled "end effectors, systems, and method for impacting prosthetics guided by surgical robots."
(Patent document 2) Korean Patent No. 10-2254406, titled "impactor for practicing medical operation"
(Patent document 3) Korean Patent No. 10-0822471, titled "tibia impactor"

### [DISCLOSURE]

### [TECHNICAL PROBLEM]

The disclosure has been conceived based on the foregoing description, and an aspect of the disclosure is to provide an impact-reducing device for medical tools, which absorbs and buffers abnormal impact applied to the medical tool, and a medical holder having the same.

### [TECHNICAL SOLUTION]

According to an aspect of the disclosure, an impact-reducing device for medical tools includes: an impact-reducing device main body formed with a tool insertion hole in which a tool shaft of the medical tool is inserted; an impact absorbing member placed in the impact-reducing device main body and absorbing impact applied to the medical tool; and a separation preventing member installed in the impact-reducing device main body to prevent separation of the impact absorbing member.

The impact absorbing member may include an elastic member embedded in the impact-reducing device main body to absorb impact applied to the tool shaft.

The elastic member may include a plurality of plate springs formed convexly to come into contact with an outer surface of the tool shaft and absorb an impact force.

The separation preventing member may include a first separation preventing flange including a first connecting portion connected to a first end of the impact-reducing device main body and formed with a through hole communicating with the tool insertion hole, and a first separation preventing protrusion protruding from an inner surface of the first connecting portion and supporting a first end of the plate spring not to be separated; and a second separation preventing flange including a second connecting portion connected to a second end of the impact-reducing device main body and formed with a through hole communicating with the tool insertion hole, and a second separation preventing protrusion protruding from an inner surface of the second connecting portion and supporting a second end of the plate spring not to be separated.

Preferably, the plate spring may be structured to include fitting portions formed at opposite ends thereof, and a convex portion formed between the fitting portions and protruding convexly toward the center of the tool insertion hole.

The first and second connecting portions may be formed to have a disc shape with a fastening hole for coupling with the impact-reducing device main body.

The first and second separation preventing protrusions may protrude along the peripheries of the through hole to provide a gap into which the fitting portion is inserted and accommodated while the first and second separation preventing flanges are coupled to the impact-reducing device main body.

Preferably, the tool insertion hole of the impact-reducing device main body may be shaped corresponding to the first and second separation preventing protrusions so that the first and second separation preventing protrusions can be inserted therein, and a sealing member can be inserted in the gap.

The first and second separation preventing protrusions may include guide flat portions having a flat structure to guide movement of the fitting portions when the plate spring is compressed and stretched.

According to an embodiment of the disclosure, a medical holder, to which a medical tool is mounted, is provided with the foregoing impact-reducing device for the medical tool.

### [ADVANTAGEOUS EFFECTS]

According to the disclosure, an impact-reducing device for medical tools and a medical holder having the same are installed on a tool shaft of the medical tool, to which an impact is applied, so that an impact absorbing member can absorb the impact to perform a buffering action, thereby preventing the medical tool or peripheral devices from being deformed or damaged.

In particular, when the impact-reducing device for medical tools according to the disclosure is applied to hip joint surgery of striking an impactor to press and fit an implant into a hip joint, a convex portion of an elastic member absorbs the impact to perform a buffering action even though a hammer misses the impactor and causes a tool shaft to be obliquely displaced, thereby preventing a holder and the impactor from being deformed or damaged.

### [DESCRIPTION OF DRAWINGS]

FIG. 1 is a perspective view of an impact-reducing device for medical tools according to an embodiment of the disclosure,
FIG. 2 is an exploded perspective view of an impact-reducing device for medical tools according to an embodiment of the disclosure,
FIG. 3 is a perspective view for illustrating the internal structure of an impact-reducing device for medical tools according to an embodiment of the disclosure,
FIG. 4 is a lateral view of an impact-reducing device for medical tools according to an embodiment of the disclosure,
FIGS. 5A and 5B are partially enlarged perspective views of an impact-reducing device for medical tools according to a first embodiment of the disclosure, in which FIG. 5A is an enlarged view of an elastic member, and FIG. 5B is an enlarged view of a separation preventing member,
FIG. 6 is a partially cutaway perspective view of the impact-reducing device for medical tools according to the first embodiment of the disclosure, and
FIGS. 7A to 7c are views for illustrating the operations of the impact-reducing device for medical tools according to the first embodiment of the disclosure, in which FIG. 7A is a perspective view shown that an impact-reducing device c and an impactor i are installed in a medical holder h, FIG. 7B is a partially enlarged view for illustrating a state that a hammer normally strikes a striking portion of the impactor, and FIG. 7C is a partially enlarged view for illustrating a state that the hammer misses the striking portion of the impactor.

### [BEST MODE]

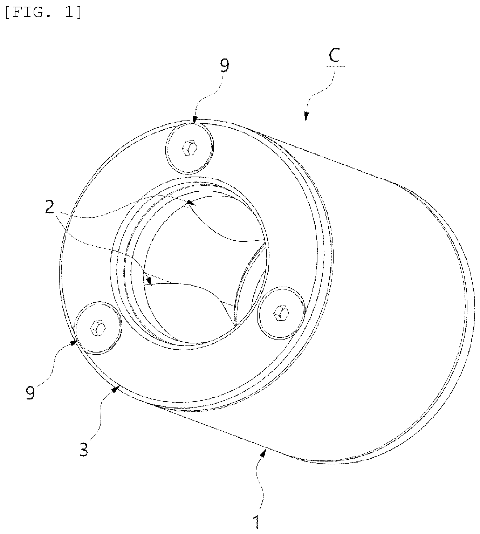

Below, exemplary embodiments of the disclosure will be described in detail with reference to FIGS. 1 to 7C, in which like numerals refer to like components throughout FIGS. 1 to 7C.

Meanwhile, detailed descriptions about components and their operations and effects, which are easily understood by a person having ordinary knowledge in the art from general technology, in the accompanying drawings will be simplified or omitted. In addition, the disclosure is characterized in an impact-reducing device for medical tools, and thus illustration and description will be made focusing on related parts while simplifying or omitting the other parts.

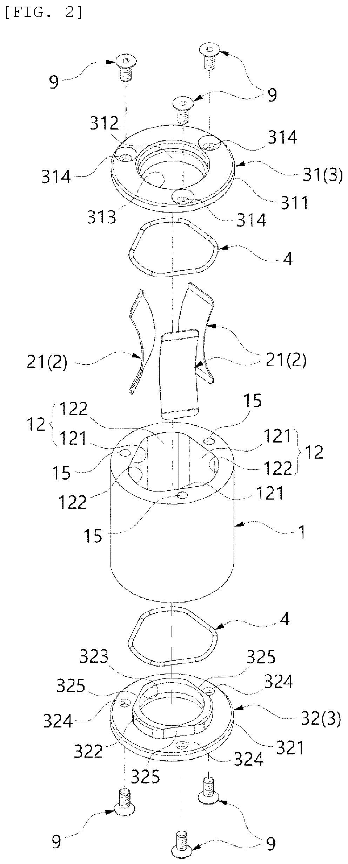

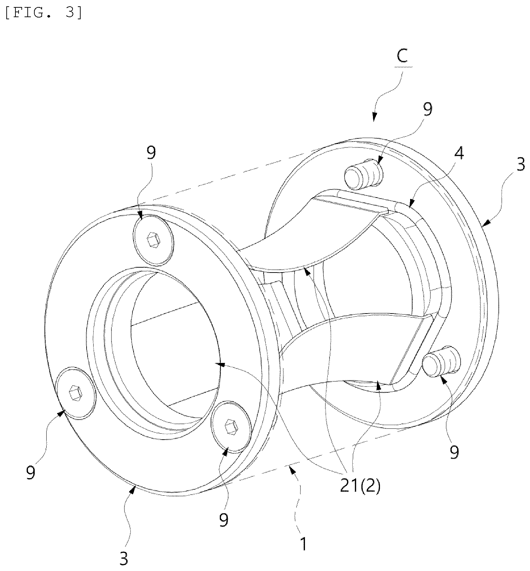

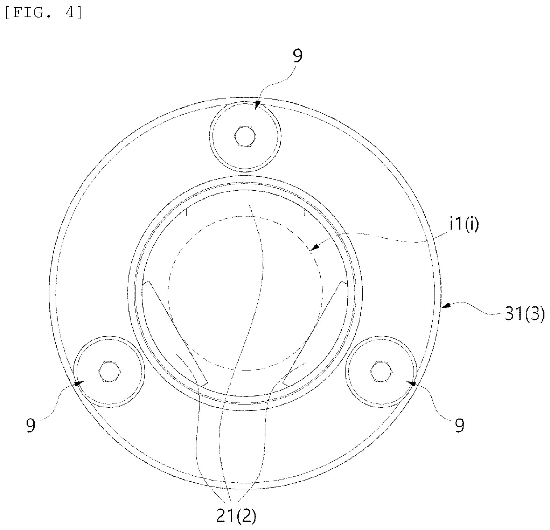

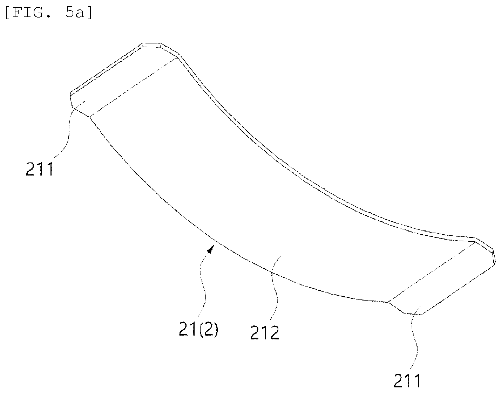

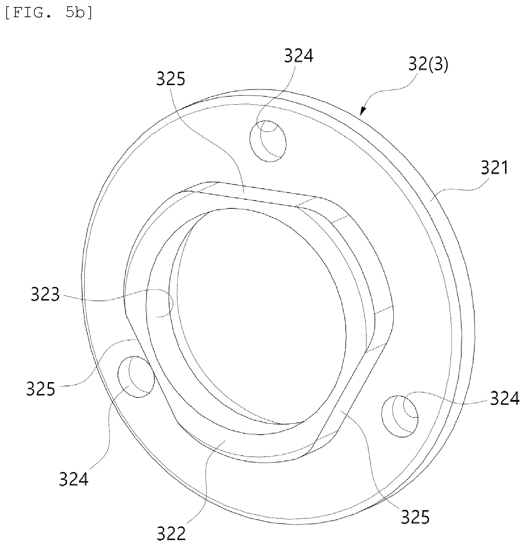

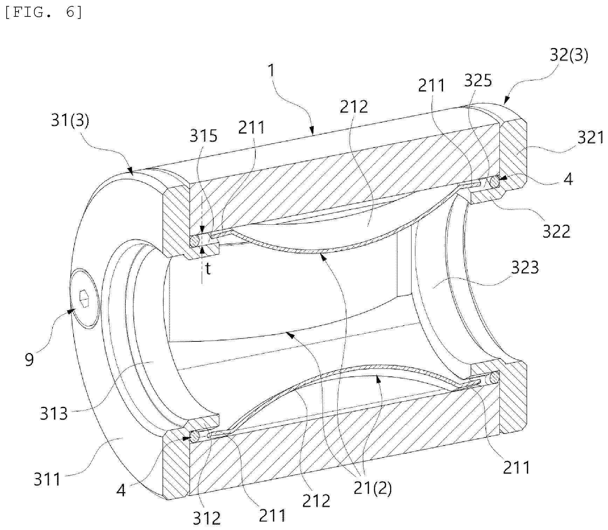

FIG. 1 is a perspective view of an impact-reducing device for medical tools according to an embodiment of the disclosure, FIG. 2 is an exploded perspective view of an impact-reducing device for medical tools according to an embodiment of the disclosure, FIG. 3 is a perspective view for illustrating the internal structure of an impact-reducing device for medical tools according to an embodiment of the disclosure, FIG. 4 is a lateral view of an impact-reducing device for medical tools according to an embodiment of the disclosure, FIGS. 5A and 5B are partially enlarged perspective views of an impact-reducing device for medical tools according to a first embodiment of the disclosure, in which FIG. 5A is an enlarged view of an elastic member, and FIG. 5B is an enlarged view of a separation preventing member, and FIG. 6 is a partially cutaway perspective view of the impact-reducing device for medical tools according to the first embodiment of the disclosure.

Referring to FIGS. 1 to 6, the impact-reducing device c for medical tools according to an embodiment of the disclosure is to prevent damage to a medical holder, a medical tool, etc. or an adverse effect on surgery by absorbing and buffering abnormal impact even though the abnormal impact is applied to the medical tool, and comprises an impact-reducing device main body 1, an impact absorbing member 2, a separation preventing member 3, and a sealing member 4.

The impact-reducing device main body 1 refers to a component, which is installed in a holder h that holds an impactor i or the like medical tool as a part of functioning as a basic framework, and is formed with a tool insertion hole 12 into which a tool shaft i1 of the medical tool is inserted.

The impact-reducing device main body 1 is approximately shaped like a cylinder and is formed with the tool insertion hole 12 into which the tool shaft i1 of the medical tool such as the impactor i is inserted.

The tool insertion hole 12 is shaped corresponding to first and second separation preventing protrusions 312 and 322 (to be described later) so that the first and second separation preventing protrusions 312 and 322 can be inserted therein, but has an inner diameter larger than an outer diameter of the first and second separation preventing protrusions 312 and 322 so as to form a gap t for the insertion of a sealing member 4 and the insertion of an elastic member 21 between the tool insertion hole 12 and the first and second separation preventing protrusions 312 and 322. For example, the tool insertion hole 12 is formed as a hole shaped like an approximately triangular prism that has three hole-flat portions 121 for inserting three plate springs, and three rounded-edge portions 122 for connecting the hole-flat portions 121.

In addition, the impact-reducing device main body 1 is formed with fastening holes 15 on the front and rear surfaces thereof being in contact with the tool insertion hole 12 so as to couple with fastening screws 9.

Meanwhile, the impact absorbing member 2 refers to a component that is placed in the impact-reducing device main body 1 and absorbs impact applied to the medical tool, and includes an elastic member 21 embedded in the impact-reducing device main body 1 to absorb the impact applied to the tool shaft i1.

The elastic member 21 may be formed to have various shapes and structures without specific limitations as long as it can absorb and buffer an oblique impact transmitted from the tool shaft i1. Considering that the tool shaft i1 in this embodiment is shaped like a round bar, the elastic member 21 includes a plurality of plate springs that are formed convexly to come into contact with the outer surface of the tool shaft i1 and absorb the impact.

Preferably, the plate spring is structured to have fitting portions 211 formed at both ends thereof, and a convex portion 212 formed between the fitting portions 211 and protruding convexly toward the center of the tool insertion hole 12, which may be formed by bending and folding a rectangular plate material. In addition, the plate spring may be manufactured by selecting any material as long as it has elasticity without specific limitations. According to this embodiment, the plate spring is manufactured using a thin metal plate of stainless steel excellent in hygiene and durability.

Meanwhile, the separation preventing member 3 refers to a component installed in the impact-reducing device main body 1 to prevent the separation of the impact absorbing member 2, and includes a first separation preventing flange 31 and a second separation preventing flange 32 connected to a first end and a second end, i.e., both ends of the impact-reducing device main body 1.

The first separation preventing flange 31 includes a first connecting portion 311 connected to the first end of the impact-reducing device main body 1 and formed with a through hole 313 communicating with the tool insertion hole 12, and a first separation preventing protrusion 312 protruding from the inner surface of the first connecting portion 311 and supporting the first end of the plate spring not to be separated.

In addition, the first connecting portion 311 is formed with the through hole 313 formed in the center of a body approximately shaped like a disc, and a plurality of fastening holes 314 equiangularly drilled for coupling with the impact-reducing device main body 1.

The second separation preventing flange 32 includes a second connecting portion 321 connected to the second end of the impact-reducing device main body 1 and formed with a through hole 323 communicating with the tool insertion hole 12 formed in the center of the body approximately shaped like a disc, and a second separation preventing protrusion 322 protruding from the inner surface of the second connecting portion 321 and supporting the second end of the plate spring not to be separated.

Like the first connecting portion 311, the second connecting portion 321 has a structure in which the through hole 323 and the plurality of fastening holes 324 are formed in a disc-shaped body.

The first and second separation preventing protrusions 312 and 322 protrude along the peripheries of the through holes 313 and 323 so as to provide the gap t into which the fitting portion 211 is inserted and accommodated while the first and second separation preventing flanges 31 and 32 are coupled to the impact-reducing device main body 1.

In particular, the first and second separation preventing protrusions 312 and 322 are characterized in including guide flat portions 315 and 325 having a flat structure to guide the movement of the fitting portions when the plate spring is compressed and stretched.

The sealing member 4 refers to a component that maintains the sealing of a connection area between the separation preventing member 3 and the impact-reducing device main body 1, and is provided as a sealing ring, i.e., a so-called O-ring.

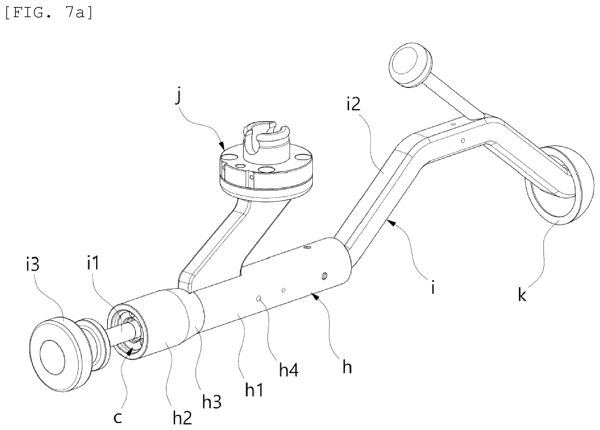

Meanwhile, the unexplained symbol i in FIG. 7A shows that the curved impactor (or curved impactor handle) is installed in the medical holder h, in which the curved impactor (or curved impactor handle) is roughly shaped like a rod and includes the tool shaft i1 having a straight structure to be inserted into the holder, a tool curved portion i2 extending toward a first side of the tool shaft i1, and a cap-shaped striking portion i3 installed at a second end of the tool shaft i1 and receiving a striking force from the hammer during surgery. Besides the foregoing curved impactor i (or curved impactor handle), an impactor used in the artificial hip joint surgery includes a straight impactor (not shown, or straight impactor handle), the outer appearance of which is shaped to have a straight structure, etc.

Further, the unexplained symbol h in FIGS. 7A to 7C shows a medical holder to which the impactor and the impact-reducing device c are mounted, and this medical holder h includes a holder body h1 approximately shaped like a pipe, an impact-reducing device insertion portion h2 having an inner diameter such that the outer diameter of the impact-reducing device c can be forcibly fitted into the front portion of the holder body h1, and a reducing portion h3 having a reduced inner diameter to prevent the impact-reducing device c being pushed rearward even when an external force is applied thereto. The holder body h1 is provided with a locking means h4 for restricting the movement of a surgical tool or supporting the surgical tool rotatably.

The unexplained symbol j shows an arm connecting member formed in the holder h and mounted to a robot arm (not shown) , and the unexplained symbol k shows an acetabulum-cup to be inserted and installed in the acetabulum of the hip joint.

Below, the operations of the impact-reducing device for medical tools according to an embodiment of the disclosure will be described in brief.

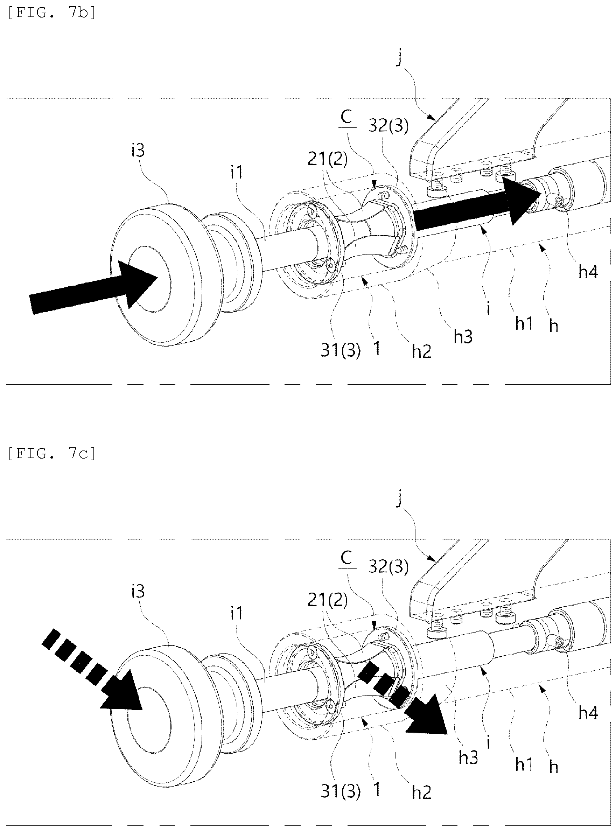

FIGS. 7A to 7c are views for illustrating the operations of the impact-reducing device for medical tools according to the first embodiment of the disclosure, in which FIG. 7A is a perspective view shown that the impact-reducing device c and the impactor i are installed in the medical holder h, FIG. 7B is a partially enlarged view for illustrating a state that a hammer normally strikes a striking portion of the impactor, and FIG. 7C is a partially enlarged view for illustrating a state that the hammer misses the striking portion of the impactor.

The process of performing the artificial hip joint surgery using the foregoing impact-reducing device c for medical tools according to the disclosure is as follows. As shown in FIG. 7A, the impact-reducing device c is installed in the impact-reducing device insertion portion h2, i.e., the front portion of the medical holder h, and the impactor i is prepared as inserted through the holder body h1. Then, the artificial acetabulum-cup k is mounted to the end of the impactor i, and inserted and installed into the acetabulum (not shown) of the hip joint by a manual surgical method or a robotic surgical method.

In more detail, the artificial hip joint surgery is usually performed by preprocessing the acetabulum of the hip joint using a reamer (not shown), inserting the artificial acetabulum-cup k into the preprocessed acetabulum, pressing and fixing the artificial acetabulum-cup k using the impactor i, and connecting an artificial femoral head (not shown) to the acetabulum-cup. Therefore, the surgical process involves striking the impactor i using the hammer (not shown).

The foregoing surgical process of striking the impactor i is performed in such a way that a user applies a striking force to the striking portion i3 using the hammer as shown in FIG. 7B. When the striking direction of the hammer is aligned with the axial direction, i.e., the longitudinal direction of the impactor, a stable surgical procedure is performed. On the other hand, when the striking direction of the hammer is misaligned with the axial direction of the impactor as shown in FIG. 7C and the impact is applied to the impactor in an oblique direction (i.e., wrong direction), the medical holder and the impactor may be deformed, damaged and broken. However, the impact-reducing device c installed in the medical holder h performs an impact absorption function, thereby preventing the foregoing problems.

In addition, the foregoing impact absorption function of the impact-reducing device c is performed in such a manner that the mishit of the hammer applies a displacement force to the tool shaft i1 in an oblique direction due to the tool shaft i1 inserted in the tool insertion hole 12 as shown in FIGS. 4 and 7c, and at the same time the convex portion 212 becomes flat to absorb and buffer the impact while the outer circumferential surface of the tool shaft i1 applies the displacement force to the elastic member 21.

Further, the impact-reducing device c for medical tools according to the disclosure may be used as an impact-absorbing means for various surgical tools having the tool shaft in addition to the medical holder h for the artificial hip joint surgery.

The terms "comprise," "configure" and/or "have" specify the presence of stated components, unless there is a clearly different meaning in the disclosure, but do not preclude the presence thereof and should be construed to further include other components. Unless otherwise defined, all terms including technical or scientific terms used herein have the same meaning as commonly understood by those skilled in the art to which the disclosure pertains. General terms that are defined in a dictionary shall be construed to have meanings that are consistent in the context of the relevant art, and will not be interpreted as having an idealistic or excessively formalistic meaning unless clearly defined in the disclosure.

Although the configurations and operations of an impact-reducing device for medical tools according to an embodiment of the disclosure and a medical holder having the same have been described so far, it will be appreciated by those skilled in the art that modifications or substitutions may be made in all or part of the embodiments of the disclosure without departing from the technical spirit of the disclosure.

Accordingly, it will be understood that the scope of the disclosure falls into the appended claims and equivalents thereof.

### [Industrial Applicability]

With an impact-reducing device for medical tools according to the disclosure and a medical holder having the same, an abnormal impact applied to the medical tool for artificial joint surgery such as artificial knee joint surgery is absorbed and buffered to prevent a medical holder, a medical tool or peripheral devices from being deformed or damaged, and the impact-reducing device may also be applied as an impact-reducing device for various medical tools or medical holders other than those for the artificial joint surgery.

## Claims

1. An impact-reducing device for medical tools, comprising:
an impact-reducing device main body formed with a tool insertion hole in which a tool shaft of the medical tool is inserted;
an impact absorbing member placed in the impact-reducing device main body and absorbing impact applied to the medical tool; and
a separation preventing member installed in the impact-reducing device main body to prevent separation of the impact absorbing member.

2. The impact-reducing device of claim 1, wherein the impact absorbing member comprises an elastic member embedded in the impact-reducing device main body to absorb impact applied to the tool shaft.

3. The impact-reducing device of claim 2, wherein the elastic member comprises a plurality of plate springs formed convexly to come into contact with an outer surface of the tool shaft and absorb an impact.

4. The impact-reducing device of claim 3, wherein the separation preventing member comprises
a first separation preventing flange comprising a first connecting portion connected to a first end of the impact-reducing device main body and formed with a through hole communicating with the tool insertion hole, and a first separation preventing protrusion protruding from an inner surface of the first connecting portion and supporting a first end of the plate spring not to be separated; and
a second separation preventing flange comprising a second connecting portion connected to a second end of the impact-reducing device main body and formed with a through hole communicating with the tool insertion hole, and a second separation preventing protrusion protruding from an inner surface of the second connecting portion and supporting a second end of the plate spring not to be separated.

5. The impact-reducing device of claim 4, wherein
the plate spring is structured to comprise fitting portions formed at opposite ends thereof, and a convex portion formed between the fitting portions and protruding convexly toward the center of the tool insertion hole,
the first and second connecting portions are formed to have a disc shape with a fastening hole for coupling with the impact-reducing device main body, and
the first and second separation preventing protrusions protrude along the peripheries of the through hole to provide a gap into which the fitting portion is inserted and accommodated while the first and second separation preventing flanges are coupled to the impact-reducing device main body.

6. The impact-reducing device of claim 5, wherein
the tool insertion hole of the impact-reducing device main body is shaped corresponding to the first and second separation preventing protrusions so that the first and second separation preventing protrusions can be inserted therein, and a sealing member can be inserted in the gap, and
the first and second separation preventing protrusions comprise guide flat portions having a flat structure to guide movement of the fitting portions when the plate spring is compressed and stretched

7. A medical holder, to which a medical tool is mounted, provided with an impact-reducing device for the medical tool according to any one of claims 1 to 6.
